# EUROPEAN PATENT APPLICATION

(11) **EP 2 631 640 A2**
(43) Date of publication of application: **28.08.2013**
(21) Application number: 13156143.3
(22) Date of filing: 21.02.2013
(51) Int. Cl.: G01N 27/72, G01N 33/40

(54) **Use of eddy currents to analyze polycrystalline diamond**

(30) Priority: 21.02.2012 US 201213401231
(71) Applicant: Varel International, Ind., L.P., Carrollton, TX 75006 (US)
(72) Inventor: Chintamaneni, Vamsee, Houston, TX Texas 77064 (US); Bellin, Federico, The Woodlands, TX Texas 77381 (US)
(74) Representative: Smee, Anthony James Michael

(57) **Abstract**

A method, system, and apparatus for non-destructively characterizing one or more regions within an ultra-hard polycrystalline structure, such as a polycrystalline diamond compact, using eddy current measurements. The apparatus includes an eddy current measuring device (41) having at least one terminal (418), a leached component (300) comprising a polycrystalline structure, a first wire (430), and a probe (440). The leached component (300) includes a cutting surface (312) and an opposing second surface (314). A portion of the polycrystalline structure extending inwardly from the cutting surface (312) has at least a portion of a catalyst material (214), such as cobalt, removed from therein. The first wire (430) electrically couples the terminal (418) to the probe (440), which is placed in contact with the cutting surface (312). The eddy current is measured one or more times and compared to a calibration curve to determine an estimated leaching depth within the polycrystalline structure. A data scattering range is ascertained to determine a relative porosity of the polycrystalline structure or the leaching quality within the polycrystalline structure.

## Description

### RELATED APPLICATIONS

The present application is related to U.S. Patent Application No. 13/401,188, entitled "Use of Capacitance to Analyze Polycrystalline Diamond" and filed on February 21, 2012, U.S. Patent Application No. 13/401,335, entitled "Use of Capacitance and Eddy Currents to Analyze Polycrystalline Diamond" and filed on February 21, 2012, and U.S. Patent Application No. 13/401,452, entitled "Method To Improve The Performance Of A Leached Cutter" and filed on February 21, 2012, which are all incorporated by reference herein.

### TECHNICAL FIELD

The present invention relates generally to a method and apparatus for measuring characteristics of one or more regions within an ultra-hard polycrystalline structure; and more particularly, to a non-destructive method and apparatus for measuring the leaching depth within the ultra-hard polycrystalline structure and/or characterizing at least a portion of the ultra-hard polycrystalline structure, such as the ones used in forming polycrystalline diamond compact ("PDC") cutters, using at least eddy current measurements.

### BACKGROUND

Polycrystalline diamond compacts ("PDC") have been used in industrial applications, including rock drilling applications and metal machining applications. Such compacts have demonstrated advantages over some other types of cutting elements, such as better wear resistance and impact resistance. The PDC can be formed by sintering individual diamond particles together under the high pressure and high temperature ("HPHT") conditions referred to as the "diamond stable region," which is typically above forty kilobars and between 1,200 degrees Celsius and 2,000 degrees Celsius, in the presence of a catalyst/solvent which promotes diamond-diamond bonding. Some examples of catalyst/solvents for sintered diamond compacts are cobalt, nickel, iron, and other Group VIII metals. PDCs usually have a diamond content greater than seventy percent by volume, with about eighty percent to about ninety-eight percent being typical. An unbacked PDC can be mechanically bonded to a tool (not shown), according to one example. Alternatively, the PDC is bonded to a substrate, thereby forming a PDC cutter, which is typically insertable within a downhole tool (not shown), such as a drill bit or a reamer.

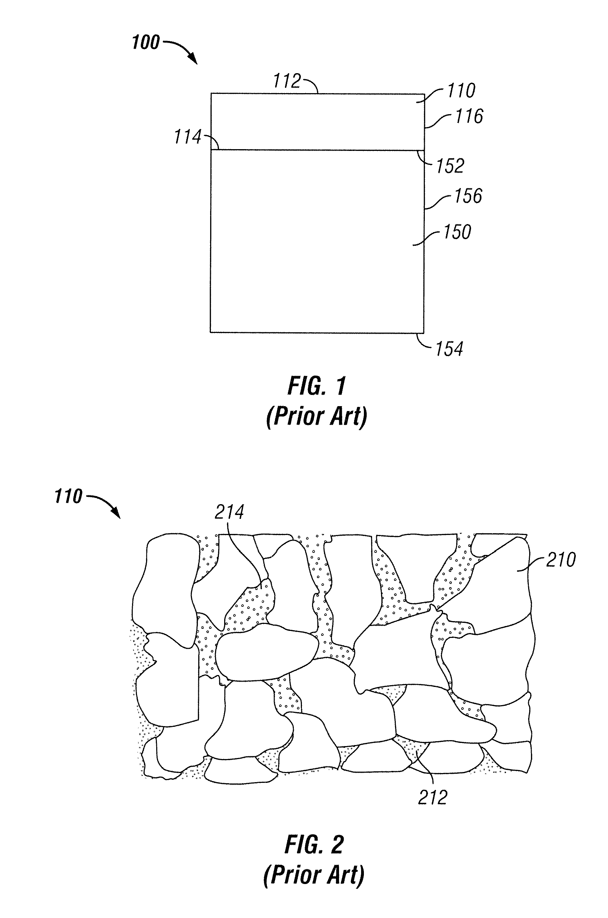

Figure 1 shows a side view of a PDC cutter 100 having a polycrystalline diamond ("PCD") cutting table 110, or compact, in accordance with the prior art. Although a PCD cutting table 110 is described in the exemplary embodiment, other types of cutting tables, including polycrystalline boron nitride ("PCBN") compacts, are used in alternative types of cutters. Referring to Figure 1, the PDC cutter 100 typically includes the PCD cutting table 110 and a substrate 150 that is coupled to the PCD cutting table 110. The PCD cutting table 110 is about one hundred thousandths of an inch (2.5 millimeters) thick; however, the thickness is variable depending upon the application in which the PCD cutting table 110 is to be used.

The substrate 150 includes a top surface 152, a bottom surface 154, and a substrate outer wall 156 that extends from the circumference of the top surface 152 to the circumference of the bottom surface 154. The PCD cutting table 110 includes a cutting surface 112, an opposing surface 114, and a PCD cutting table outer wall 116 that extends from the circumference of the cutting surface 112 to the circumference of the opposing surface 114. The opposing surface 114 of the PCD cutting table 110 is coupled to the top surface 152 of the substrate 150. Typically, the PCD cutting table 110 is coupled to the substrate 150 using a high pressure and high temperature ("HPHT") press. However, other methods known to people having ordinary skill in the art can be used to couple the PCD cutting table 110 to the substrate 150. In one embodiment, upon coupling the PCD cutting table 110 to the substrate 150, the cutting surface 112 of the PCD cutting table 110 is substantially parallel to the substrate's bottom surface 154. Additionally, the PDC cutter 100 has been illustrated as having a right circular cylindrical shape; however, the PDC cutter 100 is shaped into other geometric or non-geometric shapes in other exemplary embodiments. In certain exemplary embodiments, the opposing surface 114 and the top surface 152 are substantially planar; however, the opposing surface 114 and the top surface 152 are non-planar in other exemplary embodiments. Additionally, according to some exemplary embodiments, a bevel (not shown) is formed around at least the circumference of the cutting surface 112.

According to one example, the PDC cutter 100 is formed by independently forming the PCD cutting table 110 and the substrate 150, and thereafter bonding the PCD cutting table 110 to the substrate 150. Alternatively, the substrate 150 is initially formed and the PCD cutting table 110 is subsequently formed on the top surface 152 of the substrate 150 by placing polycrystalline diamond powder onto the top surface 152 and subjecting the polycrystalline diamond powder and the substrate 150 to a high temperature and high pressure process. Alternatively, the substrate 150 and the PCD cutting table 110 are formed and bonded together at about the same time. Although a few methods of forming the PDC cutter 100 have been briefly mentioned, other methods known to people having ordinary skill in the art can be used.

According to one example for forming the PDC cutter 100, the PCD cutting table 110 is formed and bonded to the substrate 150 by subjecting a layer of diamond powder and a mixture of tungsten carbide and cobalt powders to HPHT conditions. The cobalt is typically mixed with tungsten carbide and positioned where the substrate 150 is to be formed. The diamond powder is placed on top of the cobalt and tungsten carbide mixture and positioned where the PCD cutting table 110 is to be formed. The entire powder mixture is then subjected to HPHT conditions so that the cobalt melts and facilitates the cementing, or binding, of the tungsten carbide to form the substrate 150. The melted cobalt also diffuses, or infiltrates, into the diamond powder and acts as a catalyst for synthesizing diamond bonds and forming the PCD cutting table 110. Thus, the cobalt acts as both a binder for cementing the tungsten carbide and as a catalyst/solvent for sintering the diamond powder to form diamond-diamond bonds. The cobalt also facilitates in forming strong bonds between the PCD cutting table 110 and the cemented tungsten carbide substrate 150.

Cobalt has been a preferred constituent of the PDC manufacturing process. Traditional PDC manufacturing processes use cobalt as the binder material for forming the substrate 150 and also as the catalyst material for diamond synthesis because of the large body of knowledge related to using cobalt in these processes. The synergy between the large bodies of knowledge and the needs of the process have led to using cobalt as both the binder material and the catalyst material. However, as is known in the art, alternative metals, such as iron, nickel, chromium, manganese, and tantalum, and other suitable materials, can be used as a catalyst for diamond synthesis. When using these alternative materials as a catalyst for diamond synthesis to form the PCD cutting table 110, cobalt, or some other material such as nickel chrome or iron, is typically used as the binder material for cementing the tungsten carbide to form the substrate 150. Although some materials, such as tungsten carbide and cobalt, have been provided as examples, other materials known to people having ordinary skill in the art can be used to form the substrate 150, the PCD cutting table 110, and the bonds between the substrate 150 and the PCD cutting table 110.

Figure 2 is a schematic microstructural view of the PCD cutting table 110 of Figure 1 in accordance with the prior art. Referring to Figures 1 and 2, the PCD cutting table 110 has diamond particles 210 bonded to other diamond particles 210, one or more interstitial spaces 212 formed between the diamond particles 210, and cobalt 214, or some other catalyst, deposited within one or more of the interstitial spaces 212. During the sintering process, the interstitial spaces 212, or voids, are formed between the carbon-carbon bonds and are located between the diamond particles 210. The diffusion of cobalt 214 into the diamond powder results in cobalt 214 being deposited within these interstitial spaces 212 that are formed within the PCD cutting table 110 during the sintering process.

Once the PCD cutting table 110 is formed and placed into operation, the PCD cutting table 110 is known to wear quickly when the temperature reaches a critical temperature. This critical temperature is about 750 degrees Celsius and is reached when the PCD cutting table 110 is cutting rock formations or other known materials. The high rate of wear is believed to be caused by the differences in the thermal expansion rate between the diamond particles 210 and the cobalt 214 and also by the chemical reaction, or graphitization, that occurs between cobalt 214 and the diamond particles 210. The coefficient of thermal expansion for the diamond particles 210 is about 1.0 x 10⁻⁶ millimeters⁻¹ x Kelvin⁻¹ ("mm⁻¹K⁻¹"), while the coefficient of thermal expansion for the cobalt 214 is about 13.0 x 10⁻⁶ mm⁻¹K⁻¹. Thus, the cobalt 214 expands much faster than the diamond particles 210 at temperatures above this critical temperature, thereby making the bonds between the diamond particles 210 unstable. The PCD cutting table 110 becomes thermally degraded at temperatures above about 750 degrees Celsius and its cutting efficiency deteriorates significantly.

Efforts have been made to slow the wear of the PCD cutting table 110 at these high temperatures. These efforts include performing a leaching process on the PCD cutting table 110, which removes some of the cobalt 214 from the interstitial spaces 212. These leaching processes, which includes, but is not limited to, an acid leaching process and/or an electrolytic leaching process, is known to persons having ordinary skill in the art and is not described herein for the sake of brevity. By removing some of the cobalt 214, or catalyst, from the PCD cutting table 110, the thermal degradation of the PCD structure is reduced.

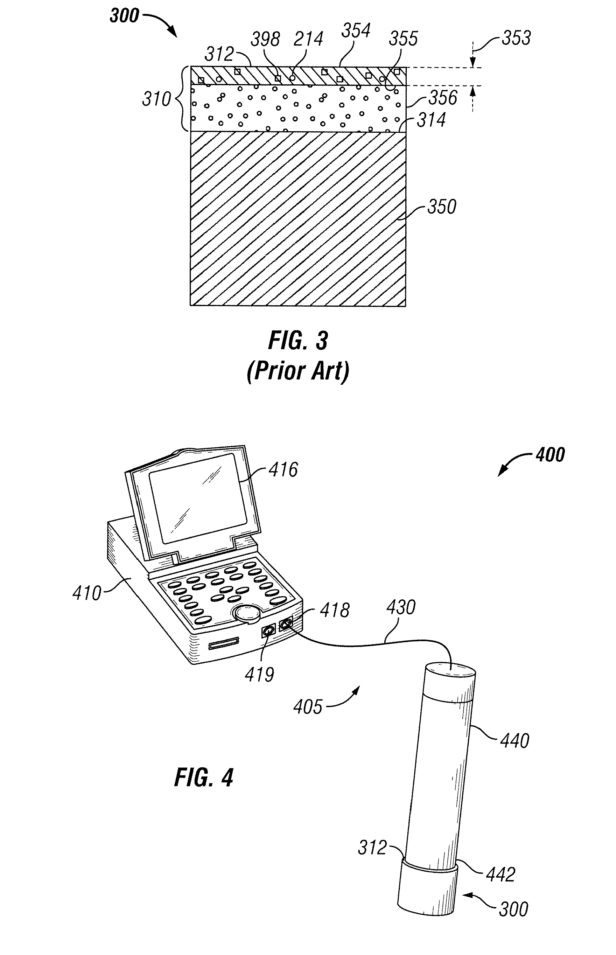

Figure 3 shows a cross-section view of a leached PDC cutter 300 having a PCD cutting table 310 that has been at least partially leached in accordance with the prior art. Referring to Figure 3, the PDC cutter 300 includes the PCD cutting table 310 coupled to a substrate 350. The substrate 350 is similar to substrate 150 (Figure 1) and is not described again for the sake of brevity. The PCD cutting table 310 is similar to the PCD cutting table 110 (Figure 1), but includes a leached layer 354 and an unleached layer 356. The leached layer 354 extends from the cutting surface 312, which is similar to the cutting surface 112 (Figure 1), towards an opposing surface 314, which is similar to the opposing surface 114 (Figure 1). In the leached layer 354, at least a portion of the cobalt 214 has been removed from within the interstitial spaces 212 (Figure 2) using at least one leaching process mentioned above. Thus, the leached layer 354 has been leached to a desired depth 353. However, during the leaching process, one or more by-product materials 398 are formed and deposited within some of the interstitial spaces 212 (Figure 2) in the leached layer 354. The unleached layer 356 is similar to the PCD cutting table 150 (Figure 1) and extends from the end of the leached layer 354 to the opposing surface 314. In the unleached layer 356, the cobalt 214 (Figure 2) remains within the interstitial spaces 212 (Figure 2). Although a boundary line 355 is formed between the leached layer 354 and the unleached layer 356 and is depicted as being substantially linear, the boundary line 355 can be non-linear.

The leached PDC cutters 300 are leached to different desired depths 353 and how deep the cutter 300 has been leached has an effect on the performance of the cutter 300. Conventionally, the leached depth 353 of the cutter 300 is measured, or determined, by cutting the cutter 300 vertically in half and then subsequently polishing the cutter 300. The leached depth 353 is visually measured under a microscope or similar magnifying device. This process is rather tedious and cumbersome as it involves cutting the cutter 300, such as by electrical discharge machining ("EDM"), mounting, grinding, and polishing the cutter 300, and performing an analysis under a microscope. Additionally, this process destroys the cutter 300 from subsequently being used. The leached depth 353 that is determined in this manner is assumed to be the same leached depth in other cutters that were leached in the same batch.

### BRIEF DESCRIPTION OF THE DRAWINGS

The foregoing and other features and aspects of the invention are best understood with reference to the following description of certain exemplary embodiments, when read in conjunction with the accompanying drawings, wherein:

Figure 1 is a side view of a PDC cutter having a polycrystalline diamond cutting table, or compact, in accordance with the prior art;

Figure 2 is a schematic microstructural view of the PCD cutting table of Figure 1 in accordance with the prior art;

Figure 3 is a cross-section view of a PDC cutter having a PCD cutting table that has been at least partially leached in accordance with the prior art;

Figure 4 is a schematic view of an eddy current testing system in accordance to one exemplary embodiment of the present invention;

Figure 5A is a graphical chart showing a plurality of shallow leached amplitude curves and a plurality of shallow leached phase angle shift curves for each of a plurality of shallow leached PDC cutters using the eddy current testing system of Figure 4 in accordance with an exemplary embodiment;

Figure 5B is a graphical chart showing a plurality of deep leached amplitude curves and a plurality of deep leached phase angle shift curves for each of a plurality of deep leached PDC cutters using the eddy current testing system of Figure 4 in accordance with an exemplary embodiment;

Figure 5C shows a graphical chart depicting both the amplitude curves and the phase angle shift curves of Figures 5A and 5B for each of the shallow leached PDC cutters and deep leached PDC cutters using the same scale in accordance with an exemplary embodiment;

Figure 6 is a flowchart depicting a non-destructive leaching depth estimation method in accordance with an exemplary embodiment of the present invention;

Figure 7A is a graphical chart depicting the calibration curve that shows a relationship between eddy current and actual leaching depth for a plurality of leached components in accordance with an exemplary embodiment of the present invention;

Figure 7B is a partial view of the graphical chart of Figure 7A in accordance with an exemplary embodiment of the present invention; and

Figure 8 is a flowchart depicting a microstructural quality determination method in accordance with an exemplary embodiment of the present invention.

The drawings illustrate only exemplary embodiments of the invention and are therefore not to be considered limiting of its scope, as the invention may admit to other equally effective embodiments.

### BRIEF DESCRIPTION OF EXEMPLARY EMBODIMENTS

The present invention is directed to a non-destructive method and apparatus for measuring the leaching depth within an ultra-hard polycrystalline structure and/or characterizing at least a portion of the ultra-hard polycrystalline structure, such as the ones used in forming polycrystalline diamond compact ("PDC") cutters, using at least eddy current measurements. Although the description of exemplary embodiments is provided below in conjunction with a PDC cutter, alternate embodiments of the invention may be applicable to other types of polycrystalline structures including, but not limited to, PCBN cutters. Further, according to some exemplary embodiments, one or more portions of the methods described below is implemented using an electronic measuring device. For example, the eddy current is measured using an eddy current measuring device. The invention is better understood by reading the following description of nonlimiting, exemplary embodiments with reference to the attached drawings, wherein like parts of each of the figures are identified by like reference characters, and which are briefly described as follows.

Figure 4 is a schematic view of an eddy current testing system 400 in accordance to one exemplary embodiment of the present invention. Referring to Figure 4, the eddy current testing system 400 includes an eddy current testing device 410, the leached PDC cutter 300, a first wire 430, and a probe 440, which collectively form a circuit 405 (described below) when electrically coupled to one another. Although certain components have been enumerated as being included in the eddy current testing system 400, additional components are included in other exemplary embodiments. Additionally, although the description provided below has been provided with respect to the leached PDC cutter 300, a different component, such as the PCD cutting table 310 alone or other component that includes another type of leached polycrystalline structure, is used in lieu of the leached PDC cutter 300. Additionally, although the description provided below has been provided with respect to the leached PDC cutter 300, a different component, such as a chemically cleaned leached PDC cutter (not shown), is used in lieu of the leached PDC cutter 300. The chemically cleaned leached PDC cutter has had at least a portion of the by-product materials 398 (Figure 3) removed by using one or more processes described in related application entitled, "Method To Improve The Performance Of A Leached Cutter", which has been mentioned above and incorporated by reference herein. The leached PDC cutter 300 has been previously described with respect to Figure 3 and is not repeated again herein for the sake of brevity.

The eddy current testing device 410 is a device that measures eddy currents, which includes at least one of an impedance amplitude and/or a phase angle shift, of the circuit 405 through which alternating current ("AC current") travels. The eddy current testing device 410 includes at least a display 416 and a first terminal 418. The display 416 is fabricated using polycarbonate, glass, plastic, or other known suitable material and communicates one or more measurement values, such as the impedance amplitude and/or the phase angle shift, to a user (not shown) of the eddy current testing device 410. The first terminal 418 is electrically coupled to one end of the first wire 430 and supplies alternating current ("AC current") therethrough. In certain exemplary embodiments, the eddy current testing device 410 includes a power supply terminal 419, which is electrically coupled to a power supply source (not shown). For example, a second wire (not shown) with a plug (not shown) electrically couples the power supply terminal 419 to a wall outlet (not shown), which is a source for alternating current. Alternatively, the eddy current testing device 410 is powered by one or more batteries (not shown) or other known power supply source. The eddy current testing device 410 displays these measurements in graphical form according to some exemplary embodiments, which can be then analyzed to determine the quantitative and/or the qualitative measurements for eddy currents. Alternatively, one or more of the impedance amplitude and/or the phase angle shift measurements are displayed digitally, or quantitatively, on the display 416.

The first wire 430 is fabricated using a copper wire or some other suitable conducting material or alloy known to people having ordinary skill in the art. According to some exemplary embodiments, the first wire 430 also includes a non-conducting sheath (not shown) that surrounds the copper wire and extends from about one end of the copper wire to an opposing end of the cooper wire. The two ends of the copper wire are exposed and are not surrounded by the non-conducting sheath. In some exemplary embodiments, an insulating material (not shown) also surrounds the copper wire and is disposed between the copper wire and the non-conducting sheath. The insulating material extends from about one end of the non-conducting sheath to about an opposing end of the non-conducting sheath. As previously mentioned, one end of the first wire 430 is electrically coupled to the first terminal 418. In certain exemplary embodiments, an adapter (not shown), or other suitable device, is coupled to the one end of the first wire 430 which also is insertable into the first terminal 418. The opposing end of the first wire 430 is electrically coupled to the probe 440.

The probe 440 includes a coil conductor (not shown) therein, which is electrically coupled to the first wire 430. According to some exemplary embodiments, the probe 440 is cylindrically shaped and includes a substantially planar first end 442 which is opposite the end at which the first wire 430 is coupled to the probe 440. However, in other exemplary embodiments, the probe 440 is shaped differently. The first end 442 is positioned in contact with the cutting surface 312 of the leached PDC cutter 300. Thus, the coil conductor transports the alternating current towards the leached PDC cutter 300.

Hence, the circuit 405 is completed using the eddy current testing device 410, the first wire 430, the probe 440, and the leached PDC cutter 300. Once the eddy current testing device 410 is turned on, the alternating current flows from the eddy current testing device 410 to the coil conductor of the probe 440 through the first wire 430. The probe 440 creates a magnetic field. The probe 440 is in close proximity to a second conductor, or the unleached layer 356 (Figure 3) of the leached PDC cutter 300, and is separated by a distance defined by the leached layer 354 (Figure 3) of the leached PDC cutter 300, which also is referred to as the leached depth 353 (Figure 3). This close proximity induces eddy currents in the unleached layer 356 (Figure 3), or second conductor, which is proportional to the frequency of the alternating current field. The distance that the coil conductor, or probe 440, is from the second conductor 356 (Figure 3), which has been mentioned above, is called liftoff. This distance affects the mutual inductance of the circuit 405 and is related to the impedance amplitude and/or a phase angle shift of the circuit 405, which is discussed in further detail below. Thus, the leached depth 353 (Figure 3) is determinable, either quantitatively and/or qualitatively, from the measured impedance amplitude and/or phase angle shift of the circuit 405. Further, the quality of leaching, i.e. the removal of catalyst material from within the leached layer 354 (Figure 3), and/or the microstructure characteristics, e.g. the porosity, is determinable, at least qualitatively, from the measured impedance amplitude and/or phase angle shift of the circuit 405. Specifically, when repeated tests on a leached PDC cutter 300 exhibits less scattered results for the measured impedance amplitude and/or phase angle shift of the circuit 405, the leached PDC cutter 300 has a better quality of leaching and/or a better microstructure characteristic.

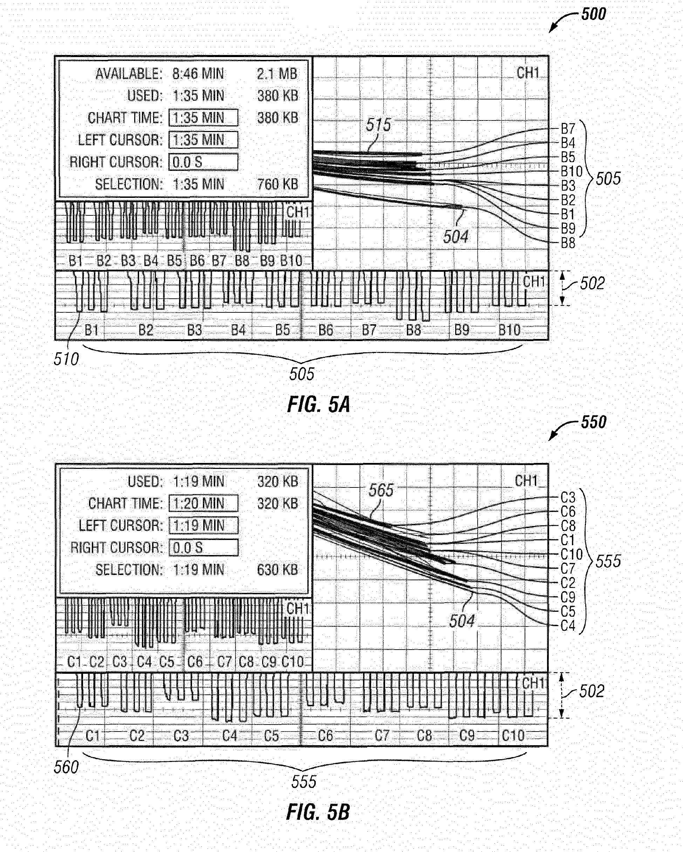

Figure 5A is a graphical chart 500 showing a plurality of shallow leached amplitude curves 510 and a plurality of shallow leached phase angle shift curves 515 for each of a plurality of shallow leached PDC cutters 505 using the eddy current testing system 400 (Figure 4) in accordance with an exemplary embodiment. The shallow leached PDC cutter 505 is similar to the leached PDC cutter 300 (Figure 3), but the leached depth 353 (Figure 3) is a shallow leached depth. A shallow leached depth typically ranges from about forty to seventy microns, but can be greater or lesser than this range in other exemplary embodiments. Referring to Figure 5A, the longitudinal length of each of the shallow leached amplitude curves 510 provides a measurement of the impedance amplitude 502 for the respective shallow leached PDC cutter 505. Similarly, the slope of each of the shallow leached phase angle shift curves 515 provides a measurement of the phase angle shift 504 for the respective shallow leached PDC cutter 505. Typically, the phase angle shift 504 is determined when analyzing the end of the shallow leached phase angle shift curve 515. The graphical chart 500 shows three shallow leached amplitude curves 510 and three shallow leached phase angle shift curves 515 for each of the ten shallow leached PDC cutters 505 that were tested using the eddy current testing system 400 (Figure 4). The impedance amplitude 502 is determined from each of the shallow leached amplitude curve 510, while the phase angle shift 504 is determined from each of the shallow leached phase angle shift curve 515.

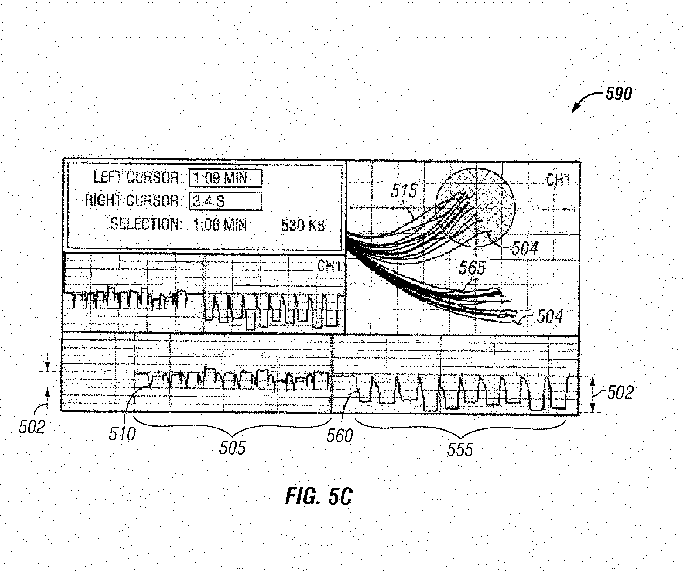

Figure 5B is a graphical chart 550 showing a plurality of deep leached amplitude curves 560 and a plurality of deep leached phase angle shift curves 565 for each of a plurality of deep leached PDC cutters 555 using the eddy current testing system 400 (Figure 4) in accordance with an exemplary embodiment. The deep leached PDC cutter 555 is similar to the leached PDC cutter 300 (Figure 3), but the leached depth 353 (Figure 3) is a deep leached depth. A deep leached depth typically ranges from about 240 to 300 microns, but can be greater or lesser than this range in other exemplary embodiments. Referring to Figure 5B, the longitudinal length of each of the deep leached amplitude curves 560 provides a measurement of the impedance amplitude 502 for the respective deep leached PDC cutter 555. Similarly, the slope of each of the deep leached phase angle shift curves 565 provides a measurement of the phase angle shift 504 for the respective deep leached PDC cutter 555. Typically, the phase angle shift 504 is determined when analyzing the end of the deep leached phase angle shift curve 565. The graphical chart 550 shows three deep leached amplitude curves 560 and three deep leached phase angle shift curves 565 for each of the ten deep leached PDC cutters 555 that were tested using the eddy current testing system 400 (Figure 4). The impedance amplitude 502 is determined from each of the deep leached amplitude curve 560, while the phase angle shift 504 is determined from each of the deep leached phase angle shift curve 565. Although Figures 5A and 5B show the test results for shallow leached PDC cutters 505 and deep leached PDC cutters 555, respectively, these test results are not comparable with one another since the scales used in depicting the amplitude curves 510, 560 and the phase angle shift curves 515, 565 are different for each of the figures. Hence, Figure 5C shows a graphical chart 590 depicting both the amplitude curves 510, 560 and the phase angle shift curves 515, 565 of Figures 5A and 5B for each of the shallow leached PDC cutters 505 and deep leached PDC cutters 555 using the same scale in accordance with an exemplary embodiment. According to Figure 5C, the impedance amplitude 502 for the shallow leached amplitude curves 510 is smaller than the impedance amplitude 502 for the deep leached amplitude curves 560. Conversely, the phase angle shift 504 for the shallow leached phase angle shift curves 515 is larger than the phase angle shift 504 for the deep leached phase angle shift curves 565.

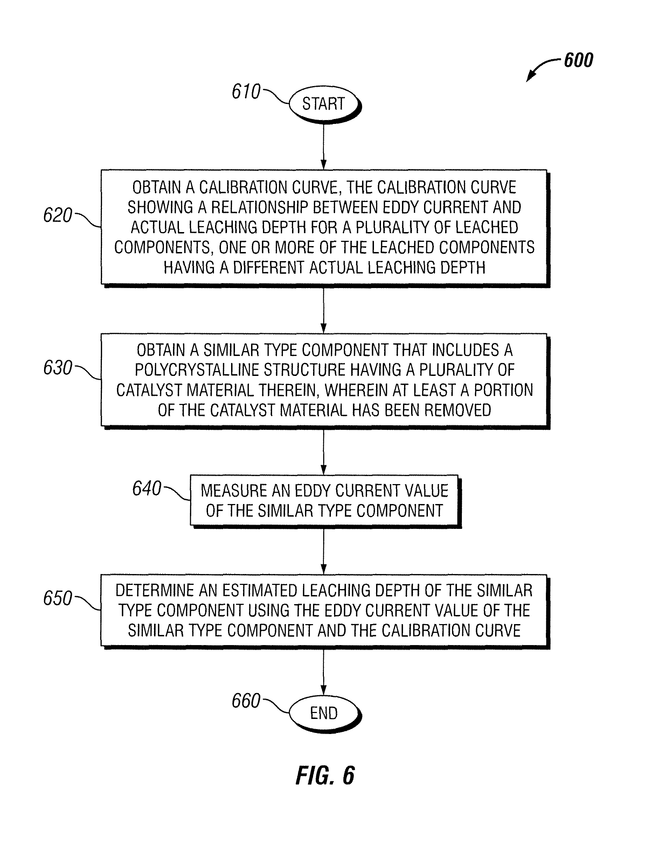

Figure 6 is a flowchart depicting a non-destructive leaching depth estimation method 600 in accordance with an exemplary embodiment of the present invention. Although Figure 6 shows a series of steps depicted in a certain order, the order of one or more steps can be rearranged, combined into fewer steps, and/or separated into more steps than that shown in other exemplary embodiments. Referring to Figure 6, the non-destructive leaching depth estimation method 600 begins at step 610. Upon starting at step 610, the non-destructive leaching depth estimation method 600 proceeds to step 620. At step 620, a calibration curve is obtained. The calibration curve can be generated from tests or acquired from elsewhere.

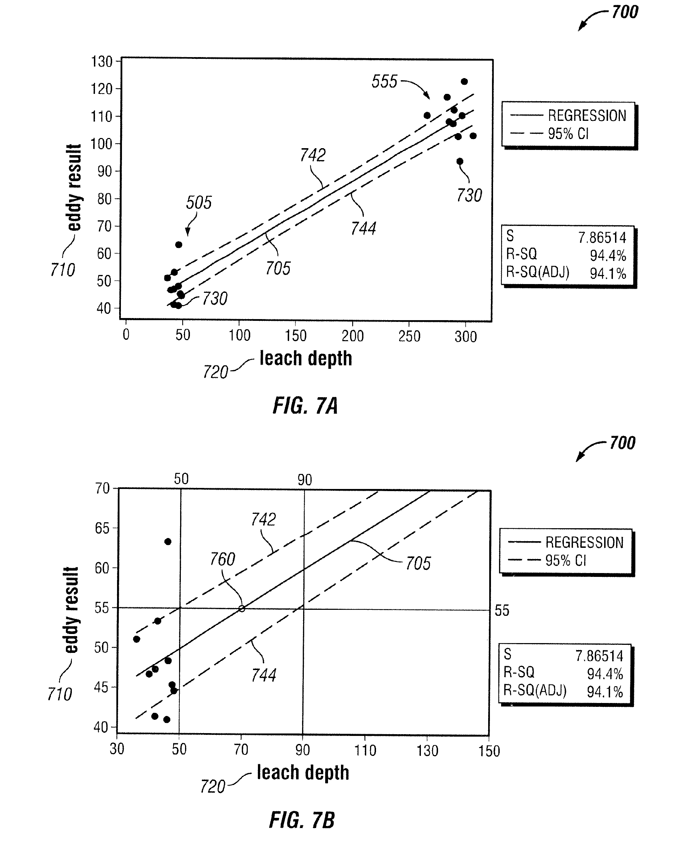

Figure 7A is a graphical chart 700 depicting the calibration curve 705 that shows a relationship between eddy current 710 and actual leaching depth 720 for a plurality of leached components 300 (Figure 3) in accordance with an exemplary embodiment of the present invention. The eddy current 710 is the impedance amplitude 502 (Figures 5A-5C) in the exemplary embodiment shown in Figure 7A, however, the phase angle shift 504 (Figures 5A-5C) can be used for the eddy current 710 in lieu of the impedance amplitude 502 (Figures 5A-5C) in other exemplary embodiments. Referring to Figure 7A, one or more of the leached components 300 (Figure 3) have a different actual leaching depth 720 than at least one other leached component 300 (Figure 3). In the exemplary embodiment shown, the leached components 300 (Figure 3) include shallow leached PDC cutters 505 and deep leached PDC cutters 555 of Figures 5A-5C. The leached component 300 (Figure 3) is the leached PDC cutter 300 (Figure 3) according to some exemplary embodiments; however, the leached component 300 can be only the PCD cutting table 310 (Figure 3) or some other component that has a polycrystalline structure that has had at least some of the catalyst material removed from therein. Alternatively, in certain exemplary embodiments, the leached component 300 can be the chemically cleaned leached PDC cutter mentioned above. Therefore, in these alternative exemplary embodiments, the leached layer 354 is treated, such as by chemical treatment, to have at least a portion of the by-product materials 398 (Figure 3) removed. This treatment is dependent upon the methods and/or chemicals used to leach the PCD cutting table 310 (Figure 3). This treated leached PDC cutter is used within the eddy current testing system 400 (Figure 4) or within some other eddy current testing system in lieu of the leached PDC cutter 300 (Figure 3) in accordance with some exemplary embodiments. In the methods using the treated leached PDC cutter, which has had at least a portion of the by-product materials 398 (Figure 3) removed, the demagnetizing step is optional.

The calibration curve 705 is generated by obtaining two or more leached components 300 (Figure 3). The calibration curve 705 becomes more precise as more leached components 300 (Figure 3) are used in generating the calibration curve 705. The eddy current data points 730 are obtained by measuring the eddy current 710, either the impedance amplitude 502 (Figures 5A-5C) and/or the phase angle shift 504 (Figures 5A-5C), of each leached component 300 (Figure 3). In certain exemplary embodiments, a plurality of eddy current data points 730 are obtained for each leached component 300 (Figure 3), which has been illustrated in Figures 5A-5C. For example, the eddy current 710 is measured three times for each leached component 300 (Figure 3). Obtaining a plurality of eddy current data points 730 for each leached component 300 (Figure 3) improves the statistical significance of the eddy current data points 730 being collected. According to some exemplary embodiments, the leached component 300 (Figure 3) is demagnetized after each measurement for eddy current 710, before each measurement for eddy current 710, or before and after each measurement for eddy current 710. The leached component 300 is demagnetized in one or a combination of different manners, such as heating the cutters above the curie temperature of the catalyst, or cobalt, which is about 1115 °C, running the cutters through an alternating current field, grounding the leached component 300 (Figure 3), wrapping the leached component 300 (Figure 3) in aluminum foil or similar type material, dropping the leached component 300 (Figure 3) in a salt solution, or waiting a period of time to discharge the leached component 300 (Figure 3). The leached component 300 (Figure 3) is demagnetized by waiting about twenty-four hours, but the waiting time is greater or less in other exemplary embodiments. Methods for demagnetizing an object is known to people having ordinary skill in the art.

Once the eddy current 710 is measured for each leached component 300 (Figure 3), the actual leaching depth 720 for each leached component 300 (Figure 3) is determined. In some examples, the actual leaching depth 720 for a leached component 300 (Figure 3) is determined by cutting the leached component 300 (Figure 3), polishing the cut edge of the leached component 300 (Figure 3), and visually measuring the actual leaching depth 720 under a magnifying device (not shown), such as a microscope. Although one method for determining the actual leaching depth 720 is described, other methods known to people having ordinary skill in the art can be used to determine the actual leaching depth 720 without departing from the scope and spirit of the exemplary embodiment. Each eddy current data point 730 is plotted on the graphical chart 700, where the actual leaching depth 720 is plotted versus the eddy current 710 that is measured. In Figure 7A, the eddy current 710 that is measured is the impedance amplitude 502 (Figures 5A-5C). Once the eddy current data points 730 are plotted on the graphical chart 700, the calibration curve 705 is determined pursuant to methods known to people having ordinary skill in the art. For example, the calibration curve 705 is generated by using the average eddy current 711 of each leached component 300 (Figure 3), the median eddy current of each leached component, or by calculating the best fit curve. The best fit curve can be formed with a ninety-five percent confidence level, but this confidence level can range from about sixty percent to almost about one hundred percent, for example, 99.99 percent.

In Figure 7A, an upper 95% confidence line 742 and a lower 95% confidence line 744 are illustrated above and below the calibration curve 705. These upper 95% confidence line 742 and lower 95% confidence line 744 provide a range for estimating the actual leaching depth 720 from the measured eddy current 710 at a 95% confidence level, which is discussed in further detail with respect to Figure 7B below. The calibration curve 705 correlates the measured eddy current 710 with the actual leaching depth 720, which can be measured in microns. Although a few methods for generating the calibration curve 705 have been described, other methods, either destructive or non-destructive, can be used to generate the calibration curve 705.

According to Figure 7A, the actual leaching depth 720 is directly related to the impedance amplitude 502, which is labeled as eddy current 710 in Figure 7A. Thus, as the actual leaching depth 720 increases, the eddy current 710, or impedance amplitude 502, that is measured also increases. Conversely, as the actual leaching depth 720 decreases, the eddy current 710, or impedance amplitude 502, that is measured also decreases. Although a direct relationship exists between the actual leaching depth 720 and the impedance amplitude 502, conversely, an indirect relationship exists between the actual leaching depth 720 and the phase angle shift.

Referring back to Figure 6, the non-destructive leaching depth estimation method 600 proceeds to step 630. At step 630, a similar type component, similar to leached cutter 300, is obtained. However, if the calibration curve was determined using treated leached PDC cutters, the similar type component is a different treated leached PDC cutter where the actual leaching depth is desired to be ascertained. This similar type component includes a polycrystalline structure that has a plurality of catalyst material therein. At least a portion of this catalyst material has been removed. This removed portion has an unknown depth, which is the leaching depth. The non-destructive leaching depth estimation method 600 proceeds to step 640. At step 640, the eddy current of the similar type component is measured. According to some exemplary embodiments, this eddy current is measured using the eddy current testing system 400 (Figure 4), wherein the impedance amplitude and/or the phase angle shift is obtained. The non-destructive leaching depth estimation method 600 proceeds to step 650. At step 650, the estimated leaching depth of the similar type component is determined using the eddy current of the similar type component and the calibration curve 705 (Figure 7A). The estimated leaching depth is an estimation of the actual leaching depth and ranges from about one micron to about fifty microns from the actual leaching depth.

Figure 7B is a partial view of the graphical chart 700 of Figure 7A in accordance with an exemplary embodiment of the present invention. Referring to Figure 7B, the eddy current 710 measured with the similar type component is fifty-five. The actual leaching depth 720 is obtained by finding a point 760 along the calibration curve 705 where the eddy current 710 is fifty-five. Thus, when the eddy current 710 is fifty-five, it is determined that the actual leaching depth 720 for the similar type component is seventy microns. Using the upper and lower 95% confidence lines 742 and 744, it is determined that the actual leaching depth 720 ranges from about fifty microns to about ninety microns at a 95% confidence level.

Referring back to Figure 6, the non-destructive leaching depth estimation method 600 proceeds to step 660, where the non-destructive leaching depth estimation method 600 ends. As previously mentioned, although the eddy current measurement used in the exemplary embodiment is the impedance amplitude, the phase angle shift can be used as the eddy current measurement in other exemplary embodiments.

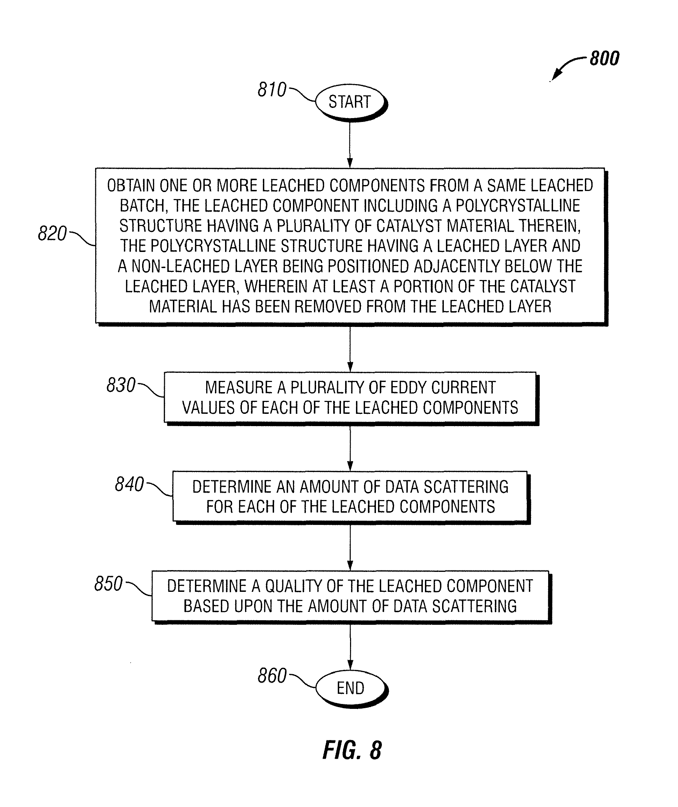

Figure 8 is a flowchart depicting a microstructural quality determination method 800 in accordance with an exemplary embodiment of the present invention. Although Figure 8 shows a series of steps depicted in a certain order, the order of one or more steps can be rearranged, combined into fewer steps, and/or separated into more steps than that shown in other exemplary embodiments. Referring to Figure 8, the microstructural quality determination method 800 begins at step 810. Upon starting at step 810, the microstructural quality determination method 800 proceeds to step 820. At step 820, one or more leached components that include a polycrystalline structure is obtained from a same leached batch. The same leached batch is a group of components that were leached in the same leaching process at the same time. The polycrystalline structure includes a leached layer and a non-leached layer being positioned adjacently below the leached layer. The non-leached layer includes a plurality of catalyst material therein, while the leached layer has had at least a portion of the catalyst material removed. The microstructural quality determination method 800 proceeds to step 830. At step 830, a plurality of eddy current values are measured for each of the leached components. The eddy current values are determined using the eddy current testing system 400 (Figure 4). The eddy current value measured is the impedance amplitude in some exemplary embodiments; however, in other exemplary embodiments, the phase angle shift is the measured eddy current value. The microstructural quality determination method 800 proceeds to step 840. At step 840, an amount of data scattering is determined for each leached component. The amount of data scattering for a leached component is determined by a differential between the highest measured eddy current value and the lowest measured eddy current value for that leached component and by statistical results of where each measured eddy current value lies. The microstructural quality determination method 800 proceeds to step 850. At step 850, a quality of the leached component is determined based upon the amount of data scattering. The quality of the leached component relates to the microstructural quality and/or the leaching quality. The microstructural quality relates to the porosity of the microstructure. The microstructural quality is a good quality when there is low porosity. Conversely, the microstructural quality is a poor quality when there is high porosity. The leaching quality is a good quality when there is less catalyst materials present within the leached layer of the polycrystalline structure. Conversely, the leaching quality is a poor quality when there is more catalyst materials present within the leached layer of the polycrystalline structure. In some exemplary embodiments, the quality of the leached component is considered to be good when the amount of data scattering is determined to be small. Conversely, the quality of the leached component is considered to be poor when the amount of data scattering is determined to be large. The relative terms of small and large are determined when comparing the data scattering of a first leached component to the data scattering of a second leached component that was leached in the same batch as the first leached component. The microstructural quality determination method 800 proceeds to step 860, where the microstructural quality determination method 800 ends.

Referring back to Figure 7A, the data scattering is seen for both shallow leached PDC cutters 505 and deep leached PDC cutters 555. Shallow leached PDC cutters 505 have less data scattering than deep leached PDC cutters 555. As shown, there are three out of ten shallow leached PDC cutters 505 that have eddy current values that fall outside of the range defined by the upper 95% confidence line 742 and the lower 95% confidence line 744, while there are six out of ten deep leached PDC cutters 555 that have eddy current values that fall outside of the range defined by the upper 95% confidence line 742 and the lower 95% confidence line 744. Hence, the shallow leached PDC cutters 505 have a better microstructural quality and/or leaching quality than the deep leached PDC cutters 555.

There are several benefits for non-destructively determining the leaching depth in an ultra-hard polycrystalline structure and/or characterizing at least a portion of the ultra-hard polycrystalline structure. For example, eddy current measurements can be made on all PDC cutters that are to be mounted and used in a tool, such as a drill bit, thereby being able to estimate the leaching depth in the ultra-hard polycrystalline structure included in the PDC cutter and/or characterizing at least a portion of the ultra-hard polycrystalline structure, such as the quality of the leaching and/or the quality of the microstructure. Hence, only certain PDC cutters are chosen to be mounted to the drill bit or other downhole tool. In another example, when a quantity of PDC cutters being leached within the same leaching batch are provided, such as one thousand PDC cutters, the eddy current of the PDC cutters are measured pursuant to the descriptions provided above. The PDC cutters that meet a desired quality and/or leaching depth are kept while the remaining PDC cutters that do not meet the desired leaching depth and/or quality are returned. Thus, in one exemplary embodiment, although one thousand PDC cutters being leached from the same batch are provided, two hundred PDC cutters, or twenty percent, may be retained while the remaining are returned. Thus, only the higher quality and/or the proper leaching depth PDC cutters are paid for and retained, which results in the PDC cutters performing better during their application.

Although each exemplary embodiment has been described in detail, it is to be construed that any features and modifications that are applicable to one embodiment are also applicable to the other embodiments. Furthermore, although the invention has been described with reference to specific embodiments, these descriptions are not meant to be construed in a limiting sense. Various modifications of the disclosed embodiments, as well as alternative embodiments of the invention will become apparent to persons of ordinary skill in the art upon reference to the description of the exemplary embodiments. It should be appreciated by those of ordinary skill in the art that the conception and the specific embodiments disclosed may be readily utilized as a basis for modifying or designing other structures or methods for carrying out the same purposes of the invention. It should also be realized by those of ordinary skill in the art that such equivalent constructions do not depart from the spirit and scope of the invention as set forth in the appended claims. It is therefore, contemplated that the claims will cover any such modifications or embodiments that fall within the scope of the invention.

The following are particularly preferred aspects according to the present disclosure.

Clause 1. An eddy current testing system, comprising: an eddy current testing device comprising at least one terminal; a leached component comprising a polycrystalline structure, the polycrystalline structure comprising a leached layer and an unleached layer positioned adjacent to the leached layer, the leached layer having at least a portion of a catalyst material removed from therein; a probe comprising a first end and a second end, the second end being placed adjacent to the surface of the leached layer; and a first wire electrically coupling the terminal of the eddy current testing device to the probe; and wherein the eddy current testing device measures an eddy current of the leached component.

Clause 2. The eddy current testing system of clause 1, wherein the eddy current of the leached component comprises an impedance amplitude.

Clause 3. The eddy current testing system of clause 1 or clause 2, wherein the eddy current of the leached component comprises a phase angle shift.

Clause 4. The eddy current testing system of any of clauses 1 to 3, wherein the leached layer has at least a portion of a by-product material removed from therein, the by-product material being formed within the leached layer during a leaching process that removes at least a portion of the catalyst material from leached layer.

Clause 5. The eddy current testing system of any of clauses 1 to 4, wherein the leached component comprises a polycrystalline diamond compact ("PDC") cutter.

Clause 6. The eddy current testing system of any of clauses 1 to 5, wherein the eddy current testing device comprises a second terminal, the second terminal being electrically coupled to a power supply source.

Clause 7. A method of characterizing a quality of a polycrystalline structure, comprising: obtaining a leached component comprising a polycrystalline structure, the polycrystalline structure comprising a leached layer and an unleached layer positioned adjacent to the leached layer, the leached layer having at least a portion of a catalyst material removed from therein; measuring at least one measured eddy current value of the leached component; and characterizing a quality of the polycrystalline structure using the at least one measured eddy current value.

Clause 8. The method of clause 7, further comprising obtaining a calibration curve showing a relationship between a plurality of eddy current values and a plurality of actual leaching depth within the polycrystalline structure, wherein characterizing a quality of the polycrystalline structure comprises using the one or more measured eddy current values of the leached component to estimate the actual leaching depth within the leached component.

Clause 9. The method of clause 7 or clause 8, further comprising obtaining a calibration curve showing a relationship between a plurality of eddy current values and a plurality of actual leaching depth within the polycrystalline structure, wherein characterizing a quality of the polycrystalline structure comprises: determining an average of the at least one measured eddy current value of the leached component; and using the average and the calibration curve to estimate the actual leaching depth within the leached component.

Clause 10. The method of any of clauses 7 to 9, further comprising demagnetizing the leached component.

Clause 11. The method of clause 10, wherein demagnetizing the leached component is performed in at least one of before measuring each measured eddy current value and after measuring each measured eddy current value.

Clause 12. The method of clause 10 or clause 11, wherein demagnetizing the leached component comprises at least one of grounding the leached component, wrapping the leached component in a demagnetizing material, heat treating the leached component, placing the leached component in a salt solution, and waiting a period of time.

Clause 13. The method of any of clause 7 to 12, further comprising cleaning the polycrystalline structure from one or more by-product materials, wherein the by-product materials were deposited within the polycrystalline structure during a leaching process that removes at least a portion of the catalyst material from therein and forms the leached component.

Clause 14. The method of any of clauses 7 to 13, further comprising:
measuring a plurality of measured eddy current values for each of a plurality of the leached components, each leached component being formed during a same leaching process, wherein characterizing a quality of the polycrystalline structure comprises using the plurality of measured eddy current values for each leached component to determine the quality of a microstructure of the polycrystalline structure for each leached component.

Clause 15. The method of clause 14, further comprising: determining a data scattering range for each of the plurality of the leached components from the plurality of measured eddy current values, wherein characterizing a quality of the polycrystalline structure comprises using the data scattering range to determine the quality of a microstructure of the polycrystalline structure for each leached component, the microstructure of the polycrystalline structure being less porous when the data scattering range is less in comparison to the data scattering ranges of other leached components.

Clause 16. The method of any of clauses 7 to 15, wherein the leached layer has at least a portion of a by-product material removed from therein, the by-product material being formed within the leached layer during a leaching process that removes at least a portion of the catalyst material from leached layer.

Clause 17. The method of any of clauses 7 to 16, wherein the measured eddy current value of the leached component comprises an impedance amplitude.

Clause 18. The method of any of clauses 7 to 17, wherein the measured eddy current value of the leached component comprises a phase angle shift.

Clause 19. The method of any of clauses 7 to 18, further comprising mounting at least a portion of the leached components to a tool based upon the characterizing of the quality of the polycrystalline structure.

Clause 20. A method of characterizing a quality of a polycrystalline structure, comprising: obtaining a leached component comprising a polycrystalline structure, the polycrystalline structure comprising a leached layer and an unleached layer positioned adjacent to the leached layer, the leached layer having at least a portion of a catalyst material removed from therein; measuring at least one measured eddy current value of the leached component; and characterizing a quality of the polycrystalline structure using the at least one measured eddy current value, the quality comprising at least one of an estimated leaching depth of the leached component, a relative amount of catalyst remaining within the leached layer, and a relative porosity of the polycrystalline structure of the leached component.

Clause 21. The method of clause 20, wherein the leached layer has at least a portion of a by-product material removed from therein, the by-product material being formed within the leached layer during a leaching process that removes at least a portion of the catalyst material from leached layer.

Clause 22. The method of clause 20 or clause 21, further comprising cleaning the polycrystalline structure from one or more by-product materials, wherein the by-product materials were deposited within the polycrystalline structure during a leaching process that removes at least a portion of the catalyst material from therein and forms the leached component.

Clause 23. The method of any of clauses 20 to 22, further comprising demagnetizing the leached component in at least one of before measuring each measured eddy current value and after measuring each measured eddy current value.

Clause 24. The method of any of clauses 20 to 23, wherein the measured eddy current value of the leached component comprises an impedance amplitude.

Clause 25. The method of any of clauses 20 to 24, wherein the measured eddy current value of the leached component comprises a phase angle shift.

Clause 26. The method of any of clauses 20 to 25, further comprising mounting at least a portion of the leached components to a tool based upon the characterizing of the quality of the polycrystalline structure.

## Claims

1. An eddy current testing system, comprising:
an eddy current testing device comprising at least one terminal;
a leached component comprising a polycrystalline structure, the polycrystalline structure comprising a leached layer and an unleached layer positioned adjacent to the leached layer, the leached layer having at least a portion of a catalyst material removed from therein;
a probe comprising a first end and a second end, the second end being placed adjacent to the surface of the leached layer; and
a first wire electrically coupling the terminal of the eddy current testing device to the probe; and
wherein the eddy current testing device measures an eddy current of the leached component.

2. The eddy current testing system of Claim 1 or Claim 2, wherein the leached component comprises a polycrystalline diamond compact ("PDC") cutter.

3. The eddy current testing system of Claim 1, wherein the eddy current testing device comprises a second terminal, the second terminal being electrically coupled to a power supply source.

4. A method of characterizing a quality of a polycrystalline structure, comprising:
obtaining a leached component comprising a polycrystalline structure, the polycrystalline structure comprising a leached layer and an unleached layer positioned adjacent to the leached layer, the leached layer having at least a portion of a catalyst material removed from therein;
measuring at least one measured eddy current value of the leached component; and
characterizing a quality of the polycrystalline structure using the at least one measured eddy current value.

5. The method of Claim 4, further comprising obtaining a calibration curve showing a relationship between a plurality of eddy current values and a plurality of actual leaching depth within the polycrystalline structure,
wherein characterizing a quality of the polycrystalline structure comprises using the one or more measured eddy current values of the leached component to estimate the actual leaching depth within the leached component.

6. The method of Claim 4 or Claim 5, further comprising obtaining a calibration curve showing a relationship between a plurality of eddy current values and a plurality of actual leaching depth within the polycrystalline structure,
wherein characterizing a quality of the polycrystalline structure comprises:
determining an average of the at least one measured eddy current value of the leached component; and
using the average and the calibration curve to estimate the actual leaching depth within the leached component.

7. The method of any of Claims 4 to 6, further comprising demagnetizing the leached component.

8. The method of Claim 7, wherein demagnetizing the leached component is performed in at least one of before measuring each measured eddy current value and after measuring each measured eddy current value.

9. The method of Claim 7 or Claim 8, wherein demagnetizing the leached component comprises at least one of grounding the leached component, wrapping the leached component in a demagnetizing material, heat treating the leached component, placing the leached component in a salt solution, and waiting a period of time.

10. The method of any of Claims 4 to 9, further comprising cleaning the polycrystalline structure from one or more by-product materials, wherein the by-product materials were deposited within the polycrystalline structure during a leaching process that removes at least a portion of the catalyst material from therein and forms the leached component.

11. The method of any of Claims 4 to 10, further comprising:
measuring a plurality of measured eddy current values for each of a plurality of the leached components, each leached component being formed during a same leaching process,
wherein characterizing a quality of the polycrystalline structure comprises using the plurality of measured eddy current values for each leached component to determine the quality of a microstructure of the polycrystalline structure for each leached component.

12. The method of Claim 11, further comprising:
determining a data scattering range for each of the plurality of the leached components from the plurality of measured eddy current values,
wherein characterizing a quality of the polycrystalline structure comprises using the data scattering range to determine the quality of a microstructure of the polycrystalline structure for each leached component, the microstructure of the polycrystalline structure being less porous when the data scattering range is less in comparison to the data scattering ranges of other leached components.

13. The eddy current testing system of any of Claims 1 to 3 or the method of any of Claims 4 to 12, wherein the leached layer has at least a portion of a by-product material removed from therein, the by-product material being formed within the leached layer during a leaching process that removes at least a portion of the catalyst material from leached layer.

14. The eddy current testing system of any of Claims 1 to 3 or the method of any of Claims 4 to 12, wherein the eddy current value of the leached component comprises an impedance amplitude.

15. The eddy current testing system of any of Claims 1 to 3 or the method of any of Claims 4 to 12, wherein the eddy current value of the leached component comprises a phase angle shift.

16. The method of any of Claims 4 to 12, further comprising mounting at least a portion of the leached components to a tool based upon the characterizing of the quality of the polycrystalline structure.
